# EUROPEAN PATENT APPLICATION

(11) **EP 4 074 365 A1**
(43) Date of publication of application: **19.10.2022**
(21) Application number: 21305511.4
(22) Date of filing: 16.04.2021
(51) Int. Cl.: A61M 60/174, A61F 2/95, A61M 60/216, A61M 60/81, A61M 60/857, A61M 60/861, A61M 60/865

(54) **SUPPORT FOR ASSIST DEVICE**

(71) Applicant: HTC-Assistance, 92110 Clichy (FR)
(72) Inventor: HELLUY, Océane-Aurore, 92110 Clichy (FR); MOUSSAFEUR, Amina, 92110 Clichy (FR); NICOL, Aurélien, 92110 Clichy (FR); SAMI, Anudeep, 92110 Clichy (FR)
(74) Representative: Icosa

(57) **Abstract**

The present invention relates to a support system (10) aimed at securing an assist device (16) inside a patient's heart, the assist device (10) comprising a pump (18) inside a pump body (20), and being in fluidic communication with an exit cylinder (24). It comprises an upstream support element (12), aimed at securing the upstream extremity (20a) of the pump body (20) to the patient's heart, a downstream support element (14), aimed at securing the exit cylinder (24) to the patient's heart. The upstream support element (12) and the downstream support element (14) are two independent and autonomous functional elements, and the upstream support element (12) and the downstream support element (14) are aimed to removably secure the assist device (16) inside the patient's heart.

## Description

### FIELD OF INVENTION

The present invention relates to means of safely and removably securing a assist device inside a patient's heart.

### BACKGROUND OF INVENTION

Heart failure remains a major health problem, with an estimated prevalence of 1-2% in the adult population of developed countries increasing to 10% from the age of 70 years.

Nowadays most cardiac assist devices are implanted in the left ventricle as most of the past years research was oriented towards it. The left ventricle has been considered, for a very long time, as the only active part of the heart, as it is bigger and more powerful than the right ventricle. As a result, the right ventricle has been considered, up to recently, as a passive reservoir and was not considered significant enough to be included in the heart assist devices research. Therefore, the few assist devices already existing for the right side of the heart are all temporary and they are mostly outside the heart or the body, requiring immobility of patients.

Most patients suffering from right ventricle dysfunction actually suffer from bi-ventricular dysfunctions, as most of the right ventricle dysfunctions are a long-term consequence from left ventricle dysfunctions. Although they affect only a small number of heart failure patients, bi-ventricular dysfunctions are a major therapeutic challenge due to its appalling prognosis. Thus, today, the reference treatment for irreversible bi-ventricular dysfunctions remains heart transplantation. However, the criteria for eligibility for the transplant (selection of candidates) and the shortage of grafts make this therapy available for only a few selected patients. In addition, the waiting lists are very long, leading thus to long waiting times, which incompatible with the precarious health of some candidates.

One way of overcoming this lack of grafts, some systems have been developed based on the combination of Left Ventricular Assis Device (LVAD) and Right Ventricular Assis Device (RVAD) leaving in place the native heart and assisting the two ventricles by two external chambers. Although it is the most usable device in clinical practice, it remains subject to a major risk of complication (infectious, thromboembolic) and the complexity of the installation will increase the mortality risk during surgery.

As an alternative treatment, the development of total artificial hearts has gradually emerged, with the issue of allowing a return home under the cover of a good quality of life. However, there are many drawbacks that limit their development, including ergonomic limitations, heavy surgery, and the instantaneous death of the patient in the event of a pump stops.

In addition, most of those patients having bi-ventricular dysfunctions are aged patients and have severe comorbidities and/or diseases, and in this case, no reasonable therapeutic solution is available, as heavy open-heart surgery is excluded.

Despite these engineering difficulties, some actors have described some clinical cases and small series using two implanted LVADs for bi-ventricular failures. Even if the idea seems attractive, the combination of two LVADs potentially exposes to a double risk of complications (infectious, surgical...) and by the limiting anatomical and physiological constraints of the right ventricle (small size and particular shape of the cavity, thinner walls and low pressure and low systemic resistance than the left ventricle). Finally, the use of LVADs devices outside of their official indication does not appear to be the solution that will satisfy the market.

In the absence of a satisfying solution to treat terminal bi-ventricular dysfunctions, the future directions include the miniaturization of LVADs allowing their implantation by percutaneous or mini-invasive access and the development of a right cardiac assistance fully implantable percutaneously to limit the surgical and infectious risk could be the solution to many patients without therapeutic project.

The development of a hybrid miniaturized RVAD used as a destination therapy combinate or not with LVAD, implanted without heavy surgery is the solution aimed at to increase the number of patients to be treated, especially patients who are not eligible for heart transplant. A further benefit is to avoid a re-intervention in case of complication or pump malfunction and the possibility to replace it percutaneously, especially in frail elderly patients. However, delivering an assist device percutaneously inside the right ventricle presents some issues like the sizing of its constitutive elements, and the positioning and securing of said elements inside the patient's heart. Another point concerns the maintenance and reparation of the assist device, if required. All the elements of the assist device and especially the elements aiming at fixating the assist device inside a patient's heart, have to thus allow a withdrawal of the assist device without surgery. This point is not so easy to handle due to well-known endothelialization processes.

### SUMMARY

This invention thus relates to a support system aimed at securing an assist device inside a patient's heart, the assist device comprising a pump and a pump body with an entrance and an exit cylinder, the pump being situated inside the pump body and being in fluidic communication with the entrance and the exit cylinder, the support system comprising:
- an upstream support element, aimed at securing the upstream extremity of the pump body to the patient's heart, the upstream support element comprising at least one foldable anchoring leg aimed at anchoring the upstream support element to the patient's heart by cooperating with some patient's heart tissue, and at least one fixation means aimed at securing the upstream extremity of the pump body,
- a downstream support element, aimed at securing the exit cylinder to the patient's heart, the downstream support element displaying a general cylindrical shape with an inner diameter and an outer diameter, the inner diameter being sized to accommodate the exit cylinder in order to radially cooperate with said exit cylinder, and the outer diameter being sized to accommodate at least one tubular shaped part of the patient's heart in order to radially cooperate with said at least one tubular shaped part,
wherein the upstream support element and the downstream support element are two independent and autonomous functional elements,
wherein the upstream support element and the downstream support element are aimed to removably secure the assist device inside the patient's heart.

This way, the solution enables to reach the here-above mentioned objective. Especially, thanks to this technical solution it is possible to dismantle and extract the assist device, and support system, place new ones in the same place, if required, while avoiding a new full surgery.

The device according to the invention may comprises one or several of the following features, taken separately from each other or combined with each other:
- the upstream support element displays at least three foldable anchoring legs,
- the upstream support element displays a general circular shape, the foldable anchoring legs being regularly distributed over its circumference,
- each foldable anchoring leg is aimed at being folded outwardly,
- each foldable anchoring leg is reversibly unfoldable,
- the downstream support element displays an open design in order to allow any fluid to flow through it,
- the downstream support element is at least partly made of a meshed material,
- the downstream support element further comprises at least one smooth spike aimed at reinforcing the radial cooperation between the downstream support element and the at least one tubular shaped part of the patient's heart,
- the downstream support element is breakable along a predetermined breaking zone defined to break when submitted to a predetermined force,
- the downstream support element is radially expandable and can be expanded in a controlled way,
- the upstream support element and the downstream support element are both radially expendable elements which can be expanded in a controlled way,
- the assist device is a right ventricle assist device, the upstream support element aims at securing the pump body to the right ventricle of the patient's heart and the downstream support element aims at securing the exit cylinder in the pulmonary artery of the patient's heart.

The invention further relates to a support system delivery system comprising:
- an introducer comprising an external handle connected to a sheath divided in a proximal flexible sheath portion and a distal bendable steerable sheath portion, the distal bendable sheath portion being controlled by the external handle,
- a catheter comprising a handle connected to a proximal flexible catheter part further connected to a distal very flexible catheter part,
- a support system according to any one of the preceding claims,
wherein the introducer and the catheter are both designed to be easily introduced and navigated in a safe and controlled way through tortuous and narrow tubular lumen or tubes,
wherein the catheter is designed to be navigated through the sheath of the introducer,
wherein the distal part of the catheter is aimed at receiving either the downstream support element or the upstream support element.

The support system delivery system may further comprise a pump pusher aimed at pushing the pump inside the pump body, through the upstream support element, the catheter being further aimed at receiving the pump body.

The invention also relates to an assist device delivery method aimed at delivering an assist device and a support system according to any one of the here-above listed features and by means of a delivery system according to any one of the technical features also listed here-above, the method comprising following steps, in the order of enunciation:
- introducing the introducer inside a lumen up to the entrance of a larger cavity,
- inserting the downstream support element, in a folded configuration, inside the distal catheter part,
- introducing, through the introducer, the catheter inside the greater cavity,
- releasing and expanding the downstream support element in the larger cavity,
- retrieving the catheter,
- inserting the upstream support element inside the distal catheter part,
- introducing, through the introducer, the catheter inside the greater cavity,
- releasing the upstream element inside the larger cavity, if needed, expanding it,
- retrieving the catheter,
- inserting the pump body inside the distal catheter part,
- introducing the catheter, through the introducer, beyond or through the expanded upstream support element and at least partially through the expanded downstream support element, inside the greater cavity,
- releasing the pump body inside the greater cavity, between the upstream support and downstream support element,
- retrieving the catheter,
- pushing the pump, by means of the pump pusher, through the introducer and the upstream support element inside the pump body,
- retrieving the pump pusher and the introducer.

### BRIEF DESCRIPTION OF THE DRAWINGS

The invention will be better understood, and other aims, details, characteristics and advantages thereof will emerge more clearly on reading the detailed description which follows, of one or several embodiments of the invention given by way of illustration. Those are purely illustrative and non-limiting examples, with reference to the accompanying schematic drawings. On these drawings:
- **Figure 1** is a histogram showing a general schematic perspective view of the system according to the present invention cooperating with an assist device,
- **Figure 2a, 2b, 2c** are several schematic views, respectively from the side in perspective and in its folded configuration of the upstream support element according to the present invention,
- **Figure 3** is a schematic view of the cooperation between the upstream support element and the pump body,
- **Figure 4** is a schematic side view of the downstream support element according to the present element.
- **Figure 5** is a schematic perspective view of the pump body according to the present invention,
- **Figure 6** is a transparence schematic view of a support device according to the present invention inside a human heart,
- **Figure 7** is a schematic longitudinal view of an introducer according to the present invention,
- **Figure 8** is a schematic longitudinal view of a catheter according to the present invention.
- **Figure 9** is a schematic longitudinal view of a pump pusher according to the invention.

### DETAILED DESCRIPTION

### Support system

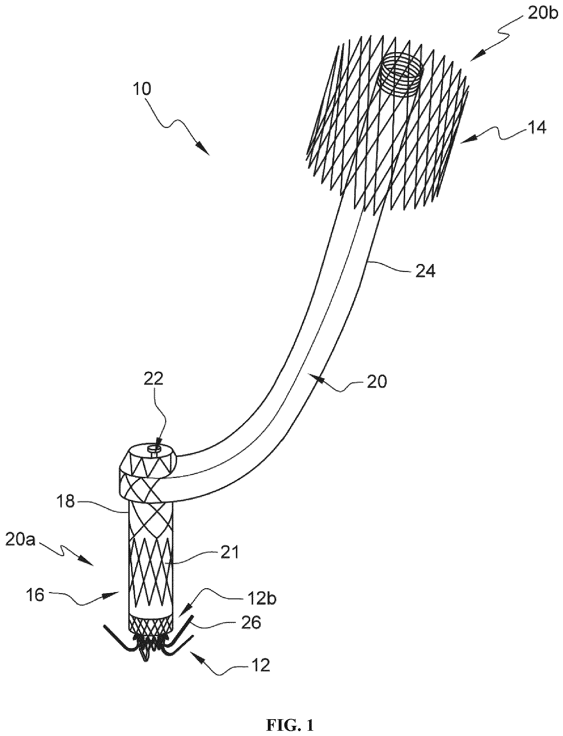

As can be seen on figure 1, the support system 10 according to the present invention, comprises two independent and autonomous stent alike functional elements: an upstream support element 12 and a downstream support element 14.

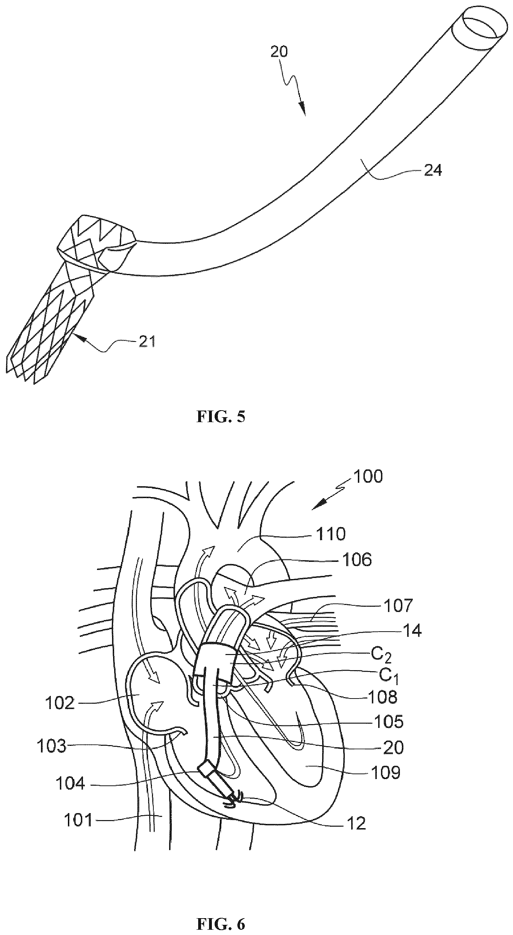

The fact that the support system 10 according to the present invention comprises two independent and autonomous functional elements 12, 14 enables significant length and diameter reductions and enables a percutaneous implantation of it. This eases the implantation process significantly. Further, the specific patient's heart 100 anatomy with its very reduced space inside the right ventricle 104 does neither allow an accurate and precise positioning nor a satisfying stability of assist the device in case the two functional elements 12, 14 could not be inserted separately. More precisely, the right ventricle 104 is a complex three-dimensional structure, with a triangular shape in sagittal cut and a crescent-shaped cross-section. Its anatomy thus makes the percutaneous implantation of any support device difficult and in order to properly position the assist device to the apex of the right ventricle while miming its natural trajectory of blood (from the right ventricle 104 to the pulmonary artery 106, see figure 6) the upstream and downstream support elements 12, 14 are designed to facilitate delivery and correctly position the assist device to accompany the blood flow. Also, the presence of two independent functional elements 12, 14 enables to safely secure the system to two different and independent anatomical parts of the patient's heart H, respectively the trabuculations of the right ventricle 104 and the pulmonary artery 106, without damaging either of them.

According to the present invention, the upstream support element 12 and the downstream support element 14 are aimed to removably secure an assist device 16 inside a patient's heart H. The advantages of the removable aspect are that, in case of a long-term assistance it may happen that the assist device has to be changed or revised. If the support elements 12, 14 of the support system 10 are removable, it facilitates the removal of the assist device itself and renders it possible without any heavy surgical intervention. The presence of two independent functional support elements 12, 14 also allows a percutaneous extraction of the system 10 without damaging the tissues of the patient's heart 100 (in accordance with the classical happening endothelialization). It thus enables a retrieval without any heavy surgery.

Regarding the present invention, the assist device 16 comprises a pump 18 and a pump body 20. The pump 18 is the rotor element of the assist device 16 which provides energy to the patient's blood in order to create flow. The created blood flow may be a continuous flow or a pulsatile flow, depending on the embodiments. More precisely, the pump 18 is the merging of a motor and a propeller (not represented). Once implanted, the pump body 20 aims at directing the blood flow generate by the pump 18 through the patient's heart 100. Moreover, it holds the pump 18 inside it in order to place the motor and the propeller of the pump in their right respective positions. For this purpose, the pump body 20 displays a cavity 21, at its upstream extremity 20a.

The pump body 20 (see figure 5) displays a general tubular shape with an upstream extremity 20a and a downstream extremity 20b. The upstream extremity 20a of the pump body 20 displays an entrance 22 aimed at letting the patient's blood flow enter the pump body 20, and the downstream extremity 20b of the pump body 20 comprises an exit cylinder 24 displaying an exit aimed at letting the patient's blood flow exit the pump body 20. The diameter of the exit cylinder 24 is about 10 to 14mm. The length of the exit cylinder 24 is comprised between 80 to 150mm. The inlet of the pump body 20 is comprised between 10 to 12mm and the pump chamber (not represented) is comprised between 12 to 16mm.

In a preferred embodiment, the pump body 20 is at least partially, preferably completely, made of meshed material, and the exit cylinder 24 can be considered as a meshed stent displaying, at least partially, a non-coating surface. This enables the pump body 20 to adopt a folded configuration or an unfolded configuration. In a functioning mode of the device 16, the pump 18 is situated inside the pump body 20 and is in fluidic communication with the entrance 22 and the exit cylinder 24. As can be seen of figure 1, the pump body 20 is not a linear tubular device and it displays, between his upstream and downstream extremities 20a, 20b, more precisely between the cavity 21 and the exit cylinder 24, a variable angle which fits the natural shape of a patient's heart 100. Since the exit cylinder 24 is made of flexible braided stent, it can adapt the patient's heart shape depending on the position of the pump body 20 inside the patient's heart 100.

The wording upstream and downstream are defined with regards to the blood flow circulation direction through the patient's heart 100 (see figure 6). Classically, the oxygen-poor blood flow coming from a patient's body is led to the patient's heart 100 by the inferior vena cava 101 and enters the patient's heart 100 through the right atrium 102. The oxygen-poor blood flow then crosses the tricuspid valve 103 and enters the right ventricle 104. The oxygen-poor blood flow then leaves the right ventricle 104 by crossing the pulmonary valve 105 and flows towards the lungs through the pulmonary artery 106. The blood flow is oxygenated in the lungs and is fled once again towards the heart 100 by the pulmonary veins 107 and reinters the heart 100 through the mitral valve 108 inside the left ventricle 109. The oxygen-rich blood flow then crosses the aortic valve 110 and leaves the heart 100 through the aorta 111 towards the patient's body.

Thus, the upstream support element 12 is aimed at securing the upstream extremity of the pump body 20 to the patient's heart 100, and the downstream support element 14 is aimed at securing the exit cylinder 24 of the pump body 20 to the patient's heart 100. In short, the upstream support element 12 aims at fixating the bottom (downstream extremity) of the pump body 20 in a location decided by an operator, and the downstream support element 14 aims at fixating the exit cylinder 24 of the pump body 20 in the right position inside and around the patient's heart 100, as will be described below.

More particularly, in a preferred embodiment (illustrated on figure 1), the device 16 is a right ventricle assist device, the upstream support element 12 aims at securing the pump body 20 to the right ventricle 104 of a patient's heart 100 and the downstream support element 14 aims at securing the exit cylinder 24 of the pump body 20 in the pulmonary artery 106 of the patient's heart 100. More precisely, in some embodiments, the downstream support element 14 and thus the exit cylinder 24 of the pump body 24 are secured to the pulmonary artery 106 through the pulmonary valve 105, blocking said pulmonary valve 105 in a permanent open configuration. In this embodiment, the pump body 20 aims at directing the blood flow generate by the pump 18 from the right ventricle 104 of the patient's heart 100 to the pulmonary artery 106.

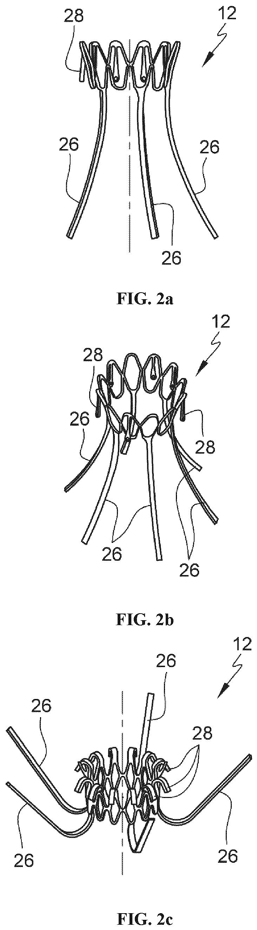

Considering now figures 2a, 2b, 2c, it appears that the upstream support element 12 comprises at least one foldable anchoring leg 26 aimed at anchoring the upstream support element 12 to the patient's heart 100 by cooperating with some patient's heart tissue. More precisely, the foldable anchoring leg 26 cooperates with the tissue of the heart wall by exerting a given pressure against said tissue, thus pushing a small part of said tissue radially outwards, and thus slightly deforming the heart wall in order to slight sink inside the heart wall. The foldable anchoring leg 26 is preferably located at the upstream extremity 12a of the upstream device 12. Each foldable anchoring leg 26 has a length comprised between 10 and 30mm.

In the embodiment illustrated on figures 1, 2a, 2b and 2c the upstream support element 12 displays at least three foldable anchoring legs 26. The upstream support element 12 further displays a general circular shape and the foldable anchoring legs 26 are regularly distributed over its circumference. In order to be able to exert pressure on the heart wall, each anchoring leg 26 is aimed at being folded outwardly. Each anchoring leg 26 is about 10 to 30mm long.

In order to facilitate the implantation of the upstream support element 12 inside a patient's heart 100, said upstream support element 12 may be radially expandable, in case it displays a circular shape. In those embodiments, the upstream support element 12 can be expanded in a controlled way. This expansion classically happens either by means of an inflatable balloon or by the use of self-expandable material. In order to allow said expansion, according to the second alternative, the upstream support element 12 has to be made, at least partially of self-expandable material like Nitinol or any other similar material. In both cases, in order to allow this expansion, and as can be seen on figures 2a, 2b and 2c, the upstream support element 12 is at least partially made of meshed material which can be folded and then unfolded, enabling the radial expansion of the upstream support element 12. In this embodiment, the upstream support element 12 may thus display a folded configuration and an expanded configuration. In this embodiment, the upstream support element 12 is made of Nitinol material.

In order to enable the removal of the pump 20 from a patient's heart 100 (detailed further below), the upstream support element 12 is removable by itself, and each foldable anchoring leg 26 is reversibly unfoldable. Each anchoring leg 26 is for example made of Nitinol.

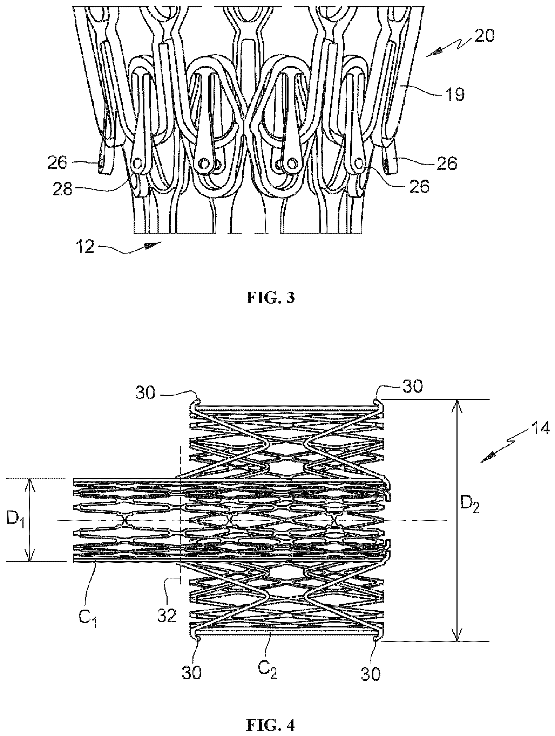

As can be seen on figure 4, the upstream support element 12 comprises a least one fixation means 28 aimed at securing the upstream extremity 20a of the pump body 20. As can be seen on figure 1, and as already mentioned, the upstream support device 12 aims at securing the cavity 21 of the pump body 20 containing the pump 18. This cavity 21 and thus the upstream extremity 20a of the pump body 20 are therefore the heaviest parts of the support system 10 and the part which is the more likely to vibrate. It is therefore really important to secure it strongly to the patient's heart 100 and to the upstream support element 12. Therefore, said fixation means 28 is preferably located on the downstream extremity 12b of the upstream support element 12. On the embodiment of figure 3, the upstream support element 12 comprises several fixations means 28 which are small elastic strips regularly distributed on the downstream extremity 12b of the upstream support element 12, each strip displaying a free end pointing in the upstream direction. This way, each strip can reversibly cooperate with the mesh of the cavity 21 of the pump body 20 and avoid any free movement within the patient's heart 100. In figure 2c, the fixation means 28 are little hooks. In some other embodiments, it might be little spikes. In any embodiment, the length of the fixations means 28 is comprised between 1 to 5mm.

Considering now the downstream support element 14, it can be seen on figure 4 that said downstream support element 14 displays a general cylindrical shape with an internal channel C₁ displaying an inner diameter D₁ and an external channel C₂ displaying an outer diameter D₂. The external channel C₂ surrounds the internal channel C₁. More particularly, the inner diameter D₁ is sized to accommodate the exit cylinder 24 of the pump body 20, and the outer diameter D₂ is sized to accommodate at least one tubular shaped part of the patient's heart 100, for example the pulmonary artery 106. The inner diameter D₁ is comprised between 8 and 15mm. The outer diameter D₂ is comprised between 26 and 32mm.

The inner diameter Di, and more particularly the wall of the internal channel Ci, thus enables the downstream support element 14 to radially cooperate with the surface of the exit cylinder 24 of the pump body 20. This radial cooperation is based on frictional forces which prevent the exit cylinder 24 from sliding along the wall of the internal channel C₁ of the downstream support element 14. The downstream support element 14 is therefore safely secured to the exit cylinder 24. In some embodiment, the wall of the internal channel C₁ displays at least one spike 25, preferably several spikes 25 regularly distributed on its circumference, said spikes 25 being able to catch the meshed non-coating surface of the exit cylinder 24, passing through the braided material of the exit cylinder 24 and thus blocking the different directions of the axial movements.

The outer diameter D₂, and more particularly the wall of the external channel C₂, thus enables the downstream support element 14 to radially cooperate, in the same way as described here-above, with the tubular shaped part of the patient's heart, for example the pulmonary artery 106. In order to reinforce the radial cooperation between the tubular shaped part of the patient's heart and the wall of the external channel C₂, the downstream support element 16 further comprises at least one smooth spike 30 with rounded edges, aimed at smoothly entering the wall tissue of the tubular shaped part of the patient's heart without damaging it, and thus anchoring the downstream support element 14 inside said wall tissue. This way, the radial force due to the outer diameter D2 expansion on the wall of tubular shaped part of the patient's heart blocks the radial movement of the downstream support element 14 and reduces its axial movement. The smooth spikes 30 deform the wall tissues of the tubular shaped part of the patient's heart in order to block the axial movement.

The result of the wall of the external channel C₂ cooperating with the tubular shaped part of the patient's heart and the wall of the internal channel C₁ cooperating with the exit cylinder 24, is that the exit cylinder 24 is safely secured inside the patient's heart 100. The downstream support element 14 thus enables to safely hold the exit cylinder 24 in position. As already detailed, the radial direction is blocked by means of the inner diameter D₁ of the downstream support element 14, which matches the exit cylinder 24 diameter. This makes sure that the exit cylinder 24 of the pump body 20 stays in place.

In order to be sure that the diameter D₂ perfectly accommodates the tubular shaped part of the patient's heart, the downstream support element 14 is radially expandable and can be expanded in a controlled way. Classically, this expansion happens inside the tubular shaped part of the patient's heart, by means of an inflatable balloon. The downstream support element 14 may thus display a folded configuration and an expanded configuration.

As the downstream support element 14 occupies the whole inner space of at least a part of the tubular shaped part of the patient's heart, it is essential that the patient's blood flow can cross it in order to exit the heart 100. The downstream support element 14 thus displays an open design in order to allow any fluid to flow through it, and more particularly, the downstream support element 14 is at least partly made of a meshed material. This meshed material also enable the downstream support element 14 to be expandable.

In the embodiment in which the assist device 16 is implanted in the right ventricle 104 of a patient's heart 100, a further technical effect of the downstream support element 14 is to center the exit cylinder 24 of the pump body 20 within the pulmonary valve 105. This centering is important because it is necessary to ensure the effective function of the pulmonary valve 105. Indeed, without the exit cylinder 24 being safely centered by means of the internal channel Ci, it would collapse on the pulmonary valve's cups and block the cups' movement or reduce their efficiency, leading to dangerous consequences. For each heart cycle, the cups of the pulmonary valve 105 coapt around the internal channel C₁ of the downstream support element 14. More particularly, the cups of the pulmonary valve 105 coapt around the upstream extremity of said internal channel C₁. The external channel C₂ is thus located downstream the cups of the pulmonary valve 105. In order to ensure good and reliable coaptation and avoid pulmonary failure, a safe distance of 5mm downstream and upstream the cups of the pulmonary valve 105 has to be respected, meaning that the downstream support element 14 has to be introduced far enough through the pulmonary valve 105 in order to distance the upstream extremity of the external channel C2 from the pulmonary valve 105.

Like the upstream support element 12, the downstream support element 14 has to allow the installation of new assist device 16 if the patient' heart 100 requires it. In order to enable the removal of the exit cylinder 24 and thus of the pump 20 from the patient's heart 100, the downstream support element 14 is breakable along a predetermined breaking zone 32 defined to break when submitted to a predetermined force. This force has to be purposely exerted by an operator. Thus, when the assist device 16 is extracted, the downstream support element 14 stays in place. This enables to avoid any damaging of the the tubular shaped part of the patient's heart due to endothelialisation (as the external channel C₂ of the downstream support element 14 is in direct contact with the wall of the tubular shaped part of the patient's heart). Thus, the downstream support element 14 is broken to allow the installation of an assist device 16.

### Support system delivery system

All the here above elements of the support system 10 are delivered inside a patient's heart 100, percutaneously, by means of a support system 10 delivery system 10' further comprising three different independent elements: an introducer 34, a catheter 36 and a pump pusher 38.

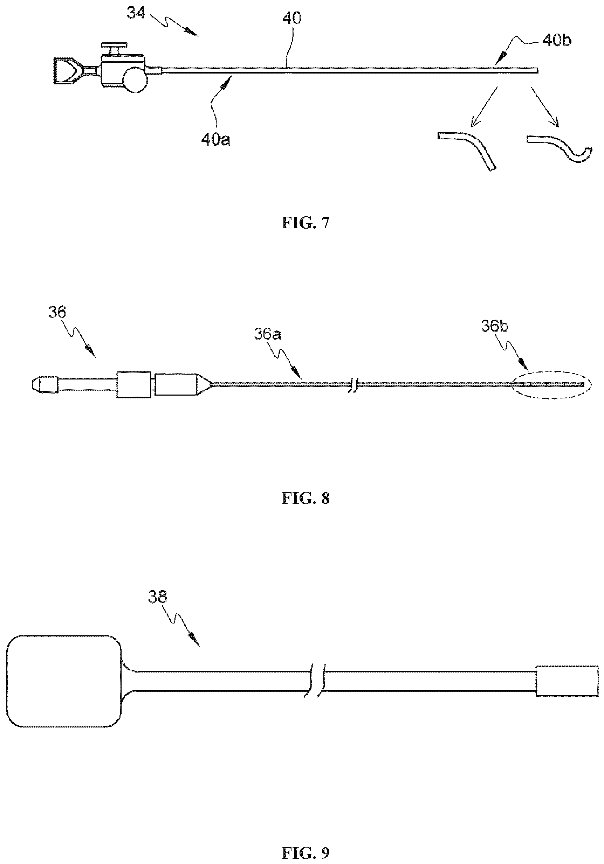

The introducer 34 comprises an external handle (see figure 7) connected to a sheath 40. The internal diameter of the sheath is from 9mm to 11.7mm with an outer diameter comprised between 10,7mmm and 13m. In some embodiments, the introducer 34 comprises an outer static sheath surrounding an inner sliding sheath. The outer static sheath reduces frictions from the inner sliding sheath when it is removed and maintains the catheter 36 in position during the delivery process. The sheath 40 is for example braided or coiled, and is divided in a proximal flexible sheath portion 40a and a distal bendable steerable sheath portion 40b. The distal bendable steerable sheath portion 40b is preferably bendable in two independent points, which can thus be bent separately. The proximal flexible sheath portion 40a enables to pass different blood vessels sinuosities of a patient. The distal bendable steerable sheath portion 40b is controlled by the external handle, and can this easily be handled by an operator from outside the patient's heart 100 and even from outside the patient's body. The bending is controlled outside by a hand wheel fixed on the external handle. The introducer 34 is used during all the delivery procedure (detailed below) and helps the catheter 36 to pass angles.

The catheter 36 also comprises a handle (see figure 8) connected to a proximal flexible catheter part 36a further connected to a distal very flexible catheter part 36b. The catheter 36 is also braided or/and coiled. The catheter 36 is designed to be navigated through the sheath 40 of the introducer 34. The catheter 36 is flexible enough to be navigate inside the blood vessels sinuosity without bending or knicking. The flexibility of the catheter 36 is different between the proximal 36a and the distal part 36b. The proximal part 36a of the catheter 36 is flexible enough to pass the small sinuosities inside internal jugular vein and the superior vena cava 101 of a patient, and also the angle made by the right atrium 102 of the patient's heart 100. Moreover, this part is navigated inside the introducer 34 until the tricuspid valve 103 during all the delivering process in order easily pass the different angles and be safely directed to the pulmonary valve 106, thanks to the distal steering of the introducer 34. The distal part 36b of the catheter 36 can receive either the downstream support element 14 or the upstream support element 12, or the pump body 20, in their folded (or crimped) configurations. The flexibility of the distal part 36b is thus increased to correctly pass the angle to reached the pulmonary valve 106 without damaging the different elements of the support system 10. The inside diameter of the catheter 36 correspond to the folded configurations of the support elements 12, 14, and the pump body 20 outer diameter, meaning between 5 to 9 mm.

The pump pusher 38 (see figure 9) is aimed at pushing the pump 18 inside the pump body 20, through the upstream support element 12, and then securing it inside.

The introducer 34 and the catheter 36 are both designed to be easily introduced and navigated in a safe and controlled way through tortuous and narrow tubular lumen or tubes, as for example human veins and arteries or a patient's heart 100.

### Delivery method

Generally speaking, the delivery method is aimed at delivering an assist device 16 and a support system 10 by means of the delivery system 10', and it comprises following steps, in the order of enunciation:
- introducing the introducer 34 inside a lumen up to the entrance of a larger cavity,
- inserting the downstream support element 14, in its folded configuration, inside the distal catheter 36b part,
- introducing, through the introducer 34, the catheter 36 inside the greater cavity,
- releasing and expanding the downstream support element 14 in the larger cavity,
- retrieving the catheter 36,
- inserting the upstream support element 12 (if needed, in its folded configuration) inside the distal catheter 36b part,
- introducing, through the introducer 34, the catheter 36 inside the greater cavity,
- releasing the upstream element 12 inside the larger cavity, if needed, expanding it,
- retrieving the catheter 36,
- inserting the pump body 20 inside the distal catheter part, preferably in its folded configuration
- introducing the catheter 36, through the introducer 34, beyond or through the expanded upstream support element 12 and at least partially through the expanded downstream support element 14, inside the greater cavity,
- releasing the pump body 20 inside the greater cavity, between the upstream support element 12 and downstream support element 14,
- retrieving the catheter 36,
- pushing the pump 18, by means of the pump pusher 38, through the introducer 34 and the upstream support element 12 inside the pump body 20,
- retrieving the pump pusher 38 and the introducer 34.

The delivery, in case it takes place in a human heart 100, is performed be a physician or a surgeon. The patient is anaesthetized locally or generally. The patient's heart 100 beats during all the delivery process. In case the delivery is an implantation inside a patient's heart 100, it is performed in four main steps because of the dimensions and shapes complexity. The delivery requires a catheter 36 for the different stent alike elements of the support system 10, an introducer 34, a balloon (not represented) and a pump pusher 38.

In case of an implantation inside a patient's heart 100, the implantation is preferably on the right side of the patient's heart 100. The path chosen for the delivery is an entrance by the internal jugular vein to reach the pulmonary valve 105 at the exit of the right ventricle 104 of a patient's heart 100. In this case, the delivery system 10' goes through the internal jugular vein, the superior vena cava 101, the right atrium 102 with the tricuspid valve 103 and the right ventricle 104. The different elements of the support system 10 and of the assist device 16 are thus implanted in the pulmonary valve 106 and the right ventricle 104.

### Preparation

At the beginning of the delivery, a guiding wire is introduced inside the internal jugular vein to guide the different elements of the delivery system 10' inside the blood vessels. Then, an introducer 34 is introduced inside the internal jugular vein. This introducer 34 is introduced up until the tricuspid valve.

### First step: Delivering of the downstream support element 14

The catheter 36 comprising the folded downstream support element 14 is introduced sliding over the guiding wire. The catheter 36 is stopped a little after the pulmonary valve 106 to deliver the downstream support element 14 in order to secure it crossing the pulmonary calve 106 and extending outwards both of its extremities. The downstream support element 14 is slowly released after checking is positioning with MRI pictures. At the end, the catheter 36 is extracted from the patient's heart 100 in sliding back over the guiding wire. A balloon is used to expand the downstream support element 14 to its unfolded configuration.

### Second step: Delivering the pump body 20

A new catheter 36 comprising the pump body 20 is inserted sliding over the guiding wire. The distal part 36b of the catheter 36 is stopped a little beyond the center of the downstream support element 14, inside the internal channel C1 to enable the fixating of the pump body to the downstream support 14. The cavity 21 of the pump body 20 is released in the right ventricle 104, totally free. The positioning is checked by RMI pictures. The catheter 36 and the guiding wire are removed from the patient's heart 100. At this step, the pump body is only fixed on his downstream extremity, to the downstream support element 14.

### Third step: Delivering the upstream support element 12

The aim of this step is to finish the fixation of the pump body 20. A new catheter 36 comprising the upstream support element 12 is introduced inside the introducer 34 following the guiding wire. After being secured to the cavity 21 of the pump body 20 the foldable legs 26 are planted inside the right ventricle 104 tissue by removing the catheter 36 a little. After checking the position of the upstream support element 12 by MRI, the catheter 36 is totally removed and the strips situated on the downstream extremity 12b catch the pump body mesh.

### Fourth and last step: Delivering the pump 18

The pump 18 is clipped to the introducer 34. With a pusher, 38 the pump 18 is put inside the introducer 34 to enter inside the body. The axial movement of the pump body 20 is blocked by the introducers' sheath 40 when the pump motor is introduced inside the pump body 20. The pusher 38 is used to apply forces on the pump 18 and place it inside the pump body. The position of the pump 18 is checked by MRI before his release. The catheter 36 and the introducer 34 are removed from the body.

In order to remove the assist device 16, the here-above steps can be followed the other way around. The only difference being that the downstream support element 14 is broken and not removed.

The present invention thus enables the delivery and maintenance of a permanent assist device inside a patient's heart, preferably in the right ventricle. The assist device is further, very easily able to be implanted and removed percutaneously without involving heavy surgery.

## Claims

1. Support system (10) aimed at securing an assist device (16) inside a patient's heart (100), the assist device (10) comprising a pump (18) and a pump body (20) with an entrance (22) and an exit cylinder (24), the pump (18) being situated inside the pump body (20) and being in fluidic communication with the entrance (22) and the exit cylinder (24), the support system (10) comprising:
- an upstream support element (12), aimed at securing the upstream extremity (20a) of the pump body (20) to the patient's heart (100), the upstream support element (12) comprising at least one foldable anchoring leg (26) aimed at anchoring the upstream support element (12) to the patient's heart (100) by cooperating with some patient's heart tissue, and at least one fixation means (28) aimed at securing the upstream extremity (20a) of the pump body (20),
- a downstream support element (14), aimed at securing the exit cylinder (24) to the patient's heart (100), the downstream support element (14) displaying a general cylindrical shape with an inner diameter (D₁) and an outer diameter (D₂), the inner diameter (D₁) being sized to accommodate the exit cylinder (24) in order to radially cooperate with said exit cylinder (24), and the outer diameter (D₂) being sized to accommodate at least one tubular shaped part of the patient's heart in order to radially cooperate with said at least one tubular shaped part,
wherein the upstream support element (12) and the downstream support element (14) are two independent and autonomous functional elements,
wherein the upstream support element (12) and the downstream support element (14) are aimed to removably secure the assist device (16) inside the patient's heart (100).

2. Support system (10) according to any one of the preceding claims, wherein the upstream support element (12) displays at least three foldable anchoring legs (26).

3. Support system (10) according to any one of the preceding claims, wherein the upstream support element (12) displays a general circular shape, the foldable anchoring legs (26) being regularly distributed over its circumference.

4. Support system (10) according to the preceding claim, wherein each foldable anchoring leg (26) is aimed at being folded outwardly.

5. Support system (10) according to any one of the preceding claims, wherein each foldable anchoring leg (26) is reversibly unfoldable.

6. Support system (10) according to any one of the preceding claims, wherein the downstream support element (14) displays an open design in order to allow any fluid to flow through it.

7. Support system (10) according to the preceding claim, wherein the downstream support element (14) is at least partly made of a meshed material.

8. Support system according to any one of the preceding claims, wherein the downstream support element (14) further comprises at least one smooth spike (30) aimed at reinforcing the radial cooperation between the downstream support element (14) and the at least one tubular shaped part of the patient's heart.

9. Support system (10) according to any one of the preceding claims, wherein the downstream support element (14) is breakable along a predetermined breaking zone (32) defined to break when submitted to a predetermined force.

10. Support system (10) according to any one of the preceding claims, wherein the downstream support element (14) is radially expandable and can be expanded in a controlled way.

11. Support system (10) according to any one of claims **3** to **10,** wherein the upstream support element (12) and the downstream support element (14) are both radially expendable elements which can be expanded in a controlled way.

12. Support system (10) according to any one of the preceding claims, wherein the assist device (16) is a right ventricle assist device (16), the upstream support element (12) aims at securing the pump body (20) to the right ventricle (104) of the patient's heart (100) and the downstream support element (14) aims at securing the exit cylinder (24) in the pulmonary artery (106) of the patient's heart (100).

13. Support system (10) delivery system (10') comprising:
- an introducer (34) comprising an external handle connected to a sheath (40) divided in a proximal flexible sheath portion (40a) and a distal bendable steerable sheath portion (40b), the distal bendable sheath portion (40b) being controlled by the external handle,
- a catheter (36) comprising a handle connected to a proximal flexible catheter part (36a) further connected to a distal very flexible catheter part (36b),
- a support system (10) according to any one of the preceding claims,
wherein the introducer (34) and the catheter (36) are both designed to be easily introduced and navigated in a safe and controlled way through tortuous and narrow tubular lumen or tubes,
wherein the catheter (36) is designed to be navigated through the sheath (40) of the introducer (34),
wherein the distal part (36b) of the catheter (36) is aimed at receiving either the downstream support element (14) or the upstream support element (12).

14. Support system (10) delivery system (10') according to the preceding claim, wherein the system (10') further comprises a pump pusher (38) aimed at pushing the pump (18) inside the pump body (20), through the upstream support element (12), the catheter (36) being further aimed at receiving the pump body (20).

15. Assist device delivery method aimed at delivering an assist device (16) and a support system (10) according to any one of claims **1** to **12** by means of a delivery system (10') according to any one of claims **13** or **14,** the method comprising following steps, in the order of enunciation:
- introducing the introducer (34) inside a lumen up to the entrance of a larger cavity,
- inserting the downstream support element (14), in a folded configuration, inside the distal catheter part (36b),
- introducing, through the introducer (34), the catheter (36) inside the greater cavity,
- releasing and expanding the downstream support element (14) in the larger cavity,
- retrieving the catheter (36),
- inserting the upstream support element (12) inside the distal catheter part (36b),
- introducing, through the introducer (34), the catheter (36) inside the greater cavity,
- releasing the upstream element inside (12) the larger cavity, if needed, expanding it,
- retrieving the catheter (36),
- inserting the pump body (20) inside the distal catheter part (36b),
- introducing the catheter (36), through the introducer (34), beyond or through the expanded upstream support element (12) and at least partially through the expanded downstream support element (14), inside the greater cavity,
- releasing the pump body (20) inside the greater cavity, between the upstream support and downstream support element (12, 14),
- retrieving the catheter (36),
- pushing the pump (18), by means of the pump pusher (38), through the introducer (34) and the upstream support element (12) inside the pump body (20),
- retrieving the pump pusher (38) and the introducer (34).
